# NEUE EUROPÄISCHE PATENTSCHRIFT

(11) **EP 0 904 059 B2**
(45) Veröffentlichungstag und Bekanntmachung des Hinweises auf die Entscheidung über den Einspruch: **19.06.2013**
(45) Hinweis auf die Patenterteilung: 18.06.2003
(21) Anmeldenummer: 97917302.8
(22) Anmeldetag: 10.04.1997
(51) Int. Cl.: A61K 9/16, A61K 9/20

(54) **GESCHMACKSVERBESSERUNG VON ARZNEIMITTELWIRKSTOFFEN**
IMPROVEMENT IN THE TASTE OF ACTIVE PHARMACEUTICAL INGREDIENTS
AMELIORATION DU GOUT DE PRINCIPES ACTIFS DE MEDICAMENTS

(30) Priorität: 02.05.1996 DE 19617487
(43) Veröffentlichungstag der Anmeldung: 31.03.1999
(73) Patentinhaber: Merck Patent GmbH, 64293 Darmstadt (DE)
(72) Erfinder: SCHWARZ, Eugen, D-64625 Bensheim (DE); MÖSCHL, Gernot, D-64331 Weiterstadt (DE); TALLAVAJHALA, Siva, Dix Hills, NY 11746 (US)
(74) Vertreter: Horstmann, Stefan
(86) Internationale Anmeldenummer: PCT/EP1997/001781
(87) Internationale Veröffentlichungsnummer: WO 1997/041835

(56) Entgegenhaltungen:
- EP-A- 0 143 576
- EP-A1- 0 275 834
- EP-A1- 0 645 096
- EP-A1- 0 834 314
- EP-A2- 0 435 450
- WO-A1-93/03633
- WO-A1-93/17667
- US-A- 3 272 704
- US-A- 4 446 135
- US-A- 4 676 984
- US-A- 4 892 889
- DATABASE WPI Section Ch, Week 9344 Derwent Publications Ltd., London, GB; Class B05, AN 93-348346 XP002042144 & JP 05 255 080 A (TAISHO PHARM.) , 5.Oktober 1993
- ROY, GLENN M.: 'Taste Masking in Oral Pharmaceuticals' PHARMACEUTICAL TECHNOLOGY Bd. 18, Nr. 4, April 1994, Seiten 84 - 99
- VOIGT, RUDOLF UNTER MITARB. V. BORNSCHEIN, MANFRED: 'Pharmazeutische Technologie für Studium und Beruf', 1995, ULLSTEIN MOSBY GMBH & CO. KG, BERLIN/WIESBADEN, ISBN 3-86126-108-1 vol. 'Tabletten', Seiten 205 - 218
- LIEBERMAN, HERBERT A., ET AL: 'Pharmaceutical Dosage Forms', Bd. 1, 1989, MARCEL DEKKER, INC., NEW YORK, 1989, ISBN 0-8247-8044-2 vol. MENDES, ROBERT W., ET AL: 'Chewable Tablets', Seiten 367 - 372

## Beschreibung

Die Erfindung betrifft ein Verfahren zur Verbesserung des Geschmacksbildes von festen Formulierungen, wie z. b. von wirkstoff- oder mineralstoffhaltigen Tabletten, und zwar sowohl des eigentlichen Geschmacks selbst als auch des sensorischen Empfindens im Mund.

Polyole und Polyolmischungen werden in großem Umfang als nicht Karies hervorrufende Zusatzstoffe und Trägerstoffe unter anderem für pharmazeutisch Wirkstoffe, Kau- und Lutschtabletten und andere Produkte der Pharmaindustrie und als Komprimate in der Lebensmittelindustrie verwendet. Gewonnen werden Polyole in der Regel durch Hydrierung der ihnen zugrundeliegenden Zucker. In fester Form können sie sowohl durch Kristallisation als auch durch Sprühtrocknung erhalten werden. Der besondere Vorteil einiger Polyole liegt darin, daß sie auch zum direkten Verpressen ohne weitere Hilfs- und Zusatzstoffe geeignet sind.

Die bekannten Polyole, Mannit, Lactit, Isomalt und Xylit sind gering hygroskopisch, zeigen jedoch schlechtes Tablettierverhalten, was sich in geringer Tablettenhärte, Deckeln und hohem Abrieb der Tabletten bemerkbar macht. Grundsätzlich werden hohe Tablettenhärten angestrebt, da in festen Formulierungen Trägerstoffe häufig nur in geringen Mengen eingesetzt werden und Wirkstoffe die Tablettenhärten drastisch vermindern können, so daß eine gegebene Rezeptur nicht tablettierbar ist.

Während Lactit, Isomalt und Xylit in der Komprimatherstellung eher ungebräuchlich sind, wird Mannit in pharmazeutischen Formulierungen durchaus verwendet.

Der Einsatz von Mannit stellt jedoch einen erhöhten Arbeitsaufwand dar, da er in der Regel vor dem Verpressen mit den übrigen Bestandteilen der Rezeptur naßgranuliert werden muß. Erhältlich ist im Handel auch direkt tablettierbarer Mannit, mit dem sich jedoch nur unbefriedigende Härten erzielen lassen, verglichen mit denen bei Verwendung von Sorbit.

Mit Sorbit, insbesondere durch Sprühtrocknung hergestellt, werden sehr gute Tablettenhärten erhalten. Gleichzeitig weisen die hergestellten Komprimate besonders glatte Oberflächen auf. Aus einer Veröffentlichung von Basedow et. al. ist bekannt, daß Calciumcarbonat-haltige Tabletten durch Verpressen mit sprühgetrocknetem oder kristallinem Sorbit hergestellt werden können. Jedoch ist die Hygroskopizität von Sorbit höherer als die anderer Polyole. Dadurch ist seine Verwendbarkeit eingeschränkt. Auch ist in diesem Zusammenhang kein Hinweis in der Literatur zu finden, daß durch Sorbit oder ein anderes Polyol das Geschmacksbild einer Formulierung wesentlich verbessert werden könnte.

Bei der Formulierung oral zu verabreichender pharmazeutischer Zusammensetzungen bereitet in vielen Fällen nicht nur bei flüssigen Applikationsformen das vom Verwender empfundene Geschmacksbild Probleme. Insbesondere beim Kauen von Antacidum-Tabletten wird ein kreidiger Geschmack als unangenehm empfunden. Es wurde bislang wenig erfolgreich durch verschiedenste Zusatzstoffe versucht, diesen kreidigen Geschmack zu überdecken. Bekannte Antacida wiederum vermitteln im Mund einen unerwünschten kreidigen, seifigen Geschmack, der durch herkömmliche Zusatzstoffe nur schwach gemildert werden kann, jedoch beim Kauen weitgehend wieder auftritt.

Als problematisch hat sich auch bei verschiedensten Wirkstoffen ein als äußerst bitter empfundener Geschmack erwiesen. Eine Maskierung besonders bitterer oder kreidiger Wirkstoffe ist bisher auch durch den Zusatz von Geschmacks- oder Aromastoffen nicht gelungen. Es besteht zwar die Möglichkeit, entsprechende Wirkstoffe enthaltende Tabletten mit einem Überzug zu versehen, diese Methode ist jedoch ungeeignet, wenn eine schnelle Aufnahme des Wirkstoffs, die bereits beim Kauen der Tabletten über die Mundschleimhaut erfolgt, angestrebt wird.

Besondere Anforderungen werden auch an die Oberfläche von Tabletten gestellt, die gelutscht werden sollen, wie beispielsweise Halstabletten. Erwünscht ist hierbei eine glatte Oberfläche der eigentlichen Tablette, die während des Lutschens erhalten bleibt und sich nicht nach und nach aufrauht.

Weiterhin werden heute im Bereich der Nahrungsergänzung (Vitamin- und Mineralstoffsupplementierung) vermehrt Lutsch- und vor allem Kautabletten angeboten. Insbesondere bei Tabletten zur Mineralstoffanreicherung ist der Trägeranteil sehr gering, so daß das Geschmacksbild weitgehend vom entsprechenden Mineralstoff geprägt ist.

Des weiteren versucht man, bei der Herstellung von festen Formulierungen zunehmend direkt verpreßbare Wirkstoffe (DC-Wirkstoffe) einzusetzen, um Produktionskosten zu senken.

Aufgabe dieser Erfindung ist es daher, ein Verfahren zur Verfügung zu stellen, wodurch einerseits das Geschmacksbild von festen Formulierungen verbessert wird, gleichzeitig aber auch das sensorische Mundgefühl der hergestellten Produkte vorteilhaft beeinflußt wird.

Es wurde nun gefunden, daß das Geschmacksbild von festen Formulierungen verbessert werden kann, indem eine einen oder mehrere Wirkstoffe enthaltende Zusammensetzung, erhältlich durch Wirbelschichtgranulation mit mindestens einem Polyol, durch Verpressen in einer festen Darreichungsform hergestellt wird.

Gegenstand der Erfindung ist somit ein Verfahren zur Herstellung von DC-Wirkstoffen, die zur Verbesserung des Geschmacksbildes von festen Formulierungen, welche einen oder mehrere Wirkstoffe enthalten, beitragen, dadurch gekennzeichnet, daß man eine einen oder mehrere Wirkstoffe enthaltende Zusammensetzung, erhältlich durch Wirbelschichtgranulation mit mindestens einem Polyol, durch Verpressen in einer festen Darreichungsform herstellt. Die eingesetzte Gesamtmenge Polyol ist dabei so zu wählen, daß in dem nach dem erfindungsgemäßen Verfahren hergestellten pulverförmige Substanzgemisch 10 bis 90 Gew.-%, insbesondere 25 bis 75 Gew.-% enthalten sind.

Die Zusammensetzung ist dabei erhältlich durch Lösen von mindestens einem Polyol in Wasser und Lösen oder Suspendieren mindestens eines Wirkstoffs in einem Lösungsmittel und Verwirbeln des erhaltenen wäßrigen Gemischs in einem Luftstrom mit einer Temperatur von 40 bis 120 °C.

Dem wäßrigen Gemisch können vor der Wirbelschichtgranulation geschmackskorrigierende Substanzen und gegebenenfalls Farbstoffe hinzugefügt sein. Als geschmackskorrigierende Substanzen kommen u. a. natürliche oder synthetische Süßstoffe aus der Gruppe Saccharin, Aspartam®, Acesulfam K, Neohesperidin DC, Sucralose, Thaumatin oder Steviosid in Frage. Als Polyole sind solche aus der Gruppe Sorbit, Mannit, Lactit, Isomalt, Maltit Erythrit oder Xylit einsetzbar. Diese können in einer Menge von 10 bis 90 Gew.-%, insbesondere 25 bis 75 Gew.-% im hergestellten Produkt enthalten sein.

Durch das erfindungsgemäße Verfahren erhält man feste Formulierungen mit verbessertem Geschmacksbild. In diesen Formulierungen können einerseits Mineralstoffe aus der Gruppe physiologisch verträglicher Ca-, Mg-, Na-, K-, Fe- und Zn-Salze in einer Menge von 0,1 bis 90 Gew.-%, insbesondere von 25 bis 75 Gew.-%, gegebenenfalls Spurenelemente, sowie ein oder mehrere Vitamin(e) und gegebenenfalls ein oder mehrere eventuell bitter schmeckende Wirkstoffe enthalten sein.

In den nach dem erfindungsgemäßen Verfahren hergestellten Formulierungen können ein oder mehrere pharmazeutische Wirkstoffe enthalten sein. Solche Wirkstoffe können unter anderem sein Antacida, Antiallergika, Analgetika, Hormone, Steroide, Östrogene, Kontrazeptiva, nasale Dekongestionsmittel, H₁- und H₂-Antagonisten, β₂-Stimulantien, Vasodilatoren, Antihypertensiva, infektionsvorbeugende Mittel, Laxantien, Antitussiva, Bronchodilatoren, Mittel gegen Halsschmerzen, Wismut und seine Salze, Pilzmittel, Antibiotika, Stimulanzien (wie z. B. Amphetamine) Alkaloide, orale Hypoglycaemica, Diuretica, Cholesterin senkende Mittel, Kombinationen verschiedener pharmazeutisch wirksamer Mittel oder andere. Diese Wirkstoffe können in einer Menge von 0,1 bis 70 Gew.-% enthalten sein.

Die Wirkstoffe können als beschichtete Teilchen, Liposomen oder Mikropartikel vorliegen. Als Beschichtung können übliche, in der Pharmazie verwendete Substanzen dienen. Die Teilchengröße der Wirkstoffe beträgt vorzugsweise 0,1 bis 800 µm mit einer Schüttdichte von 0,15 bis 1 g/cm³.

Natürlich sind die im vorhergehenden Text gegebenen Gew.-%-Angaben so zu verstehen, daß die Wahl der Gewichtsprozente der eingesetzten Substanzen in der Summe 100 nicht übersteigen.

Der Begriff Polyol steht für Zuckeralkohole der allgemeinen Formel CH₂OH-(CHOH)ₙ-CH₂OH,
wobei n für 2 bis 6, vorzugsweise 3 bis 4, steht,
sowie deren dimere Anhydride, insbesondere C₁₂H₂₄O₁₁.

Insbesondere steht der Begriff Polyole für Hexite wie Sorbit und Mannit, Pentite wie Xylit, möglich sind aber auch C₄-Polyalkohole wie Erythrit oder C₁₂-Polyalkohole wie Lactit oder Maltit. Der Begriff Polyole steht aber auch für geeignete Kohlehydrate.

Die Wirbleschichtgranulation wird, wie z. B. in P. Grassmann, F. Widmer" Einführung in die thermische Verfahrenstechnik" beschrieben, durchgeführt.

Aufgrund der besonderen Herstellungsart durch Versprühen einer wäßrigen Lösung ist es möglich, nicht wasserlösliche und wasserlösliche Zusätze, wie z. b. Zitronensäure, Süßstoffe, insbesondere Acesulfam K, Aspartam®, Saccharin, Cyclamat, Sucralose, Neohesperidin DC, Farbstoffe sowie pharmazeutische Wirkstoffe, wie z. B. Analgetika, Antacida und dergleichen, Vitamine und gegebenenfalls Spurenelemente homogen in den erfindungsgemäßen Zusammensetzungen, bzw. festen Formulierung und den daraus hergestellten Tabletten zu verteilen.

Die gegebenenfalls zuzusetzenden Bindemittel sind dem Fachmann geläufig und dienen der Erhöhung der Festigkeit der Zusammensetzung. Als Bindemittel bevorzugt sind Cellulose-Derivate, insbesondere Hydroxypropylmethylcellulose, Carboxymethylcellulose oder Stärke.

Die so charakterisierten Polyol-Zusammensetzungen besitzen eine Reihe von vorteilhaften Tablettiereigenschaften:

Überraschenderweise kann festgestellt werden, daß durch das erfindungsgemäße Verfahren feste Formulierungen, insbesondere Tabletten, mit einem erheblich verbesserten Geschmacksbild und sensorischem Mundgefühl erhalten werden. Es handelt sich dabei also um direkt verpreßbare Wirkstoffformulierungen (DC-Wirkstoffe).

Bei Verwendung von Formulierungen mit einem hohen Mineralstoffgehalt von bis zu 90 Gew.-% werden einerseits drastisch verbesserte Tablettiereigenschaften gefunden, andererseits sind die hergestellten Tabletten während des Verpackungsvorgangs durch einen wesentlich geringeren Abrieb gekennzeichnet. Auch werden bei Verwendung der erfindungsgemäßen Zusammensetzungen bei gleicher Preßkraft, wie sie bei bekannten polyolhaltigen Formulierungen angelegt wird, härtere Tabletten mit glatteren Oberflächen erhalten. Dieses anfänglich empfundene verbesserte sensorische Gefühl im Mund wird auch beim Kauen oder Lutschen empfunden, da der sonst übliche kreidige, oder gegebenenfalls seifige Geschmack weitestgehend überdeckt ist. Überraschender Weise wird jedoch nicht nur das Geschmacksbild dieser Mineralstofftabletten verbessert. Auch Formulierungen, in die äußerst bitter schmeckende Wirkstoffe eingearbeitet sind, werden als wesentlich wohlschmeckender empfunden, da der Bittergeschmack nicht mehr so extrem durchschlägt.

Die nachfolgenden Beispiele dienen der besseren Veranschaulichung der beschriebenen und beanspruchten Erfindung, sind jedoch nicht zur Einengung des Schutzbereichs auf diese Beispiele geeignet.

### Beispiele

### Beispiel 1

### Analgetikum-Tablette

Zusammensetzung des Tablettiermaterials:

| | |
|---|---|
| Acetylsalicylsäure (ASS) | 74,0 % |
| Sorbit-Anteil | 24,5 % |
| (Instant-Qualität beigemischt, bzw. mit ASS gesprüht) | |
| Acesulfam K | 0,5 % |
| Magnesiumstearat | 1,0 % |

### Tablettier- und Geschmacksvergleich

### Herstellung einer mechanischen Mischung:

ASS wird durch Mahlung fein gepulvert und mit Sorbit Instant im Turbula-Schüttel-Mischer einschließlich des mikronisierten Süßstoffes ASK verrieben. Anschließend erfolgt eine Siebung zur Desagglomeration mit einem 1 mm-Sieb und die Nachmischung mit Magnesiumstearat und anschließend die Tablettierung.

### Herstellung von co-gesprühtes Tabletten Material:

Feingepulverte, gemahlene ASS wird teilweise (ca. 5 %) in der Wirbelschicht vorgelegt. und der Rest im Verhältnis von ca. 1 : 1 in Sorbitlösung dispergiert. Die Dispersion wird durch Rühren aufrecht erhalten und mit dem Süßstoff ASK direkt in das Wirbelbett gesprüht, bei gleichzeitiger Wasserverdampfung.

Dem so gebildeten relativ feinen Sprühgranulat wird als Gleitmittel 1 % Magnesiumstearat beigemischt. Die erhaltene Mischung wird direkt tablettiert.

### Ergebnisse:

| | Mechan. Mischung | Cogesprühtes Material |
|---|---|---|
| Aussehen | wenig rieselfähige Mischung, deren beiden Hauptkomponenten erkennbar sind | homogenes, gut rieselfähiges Tablettier-Granulat |
| Tablettierung: 500 mg Preßkraft 10 kN | | |
| Tablettenhärte: | 90 N | 210 N |
| Abrieb (Friab.) | 0,6 % | 0,3 % |
| Sensorik | säuredominanter Geschmack, hohe Oberflächenrauheit der Tablette | gemilderter süßsaurer Geschmack, angenehmes Kauverhalten (glatte, wenig saugfähige Oberfläche) |

### Beispiel 2

### Vitamin C-Tablette

| | |
|---|---|
| Ascorbinsäure | 87,7 % |
| Sorbit | 10,0% |
| Orangenaroma | 0,7 % |
| (pulverförmig) | |
| Acesulfam K | 0,6 % |
| Mg-Stearat | 1,0% |

### Herstellung einer mechanischen Mischung:

Die feinkristalline Ascorbinsäure wird im Turbula-Schüttelmischer mit Sorbit Instant (Körnung unter 0,3 mm), Süßstoff und Aroma innig verrieben. Danach wird das Gleitmittel Magnesium-Stearat aufgesiebt und eingemischt.

### Herstellung von co-gesprühtem Tablettiermaterial:

Ascorbinsäure, Sorbit und Süßstoff werden mit ca. 40 % Feststoffanteil in Wasser bei 40 °C gelöst und auf ein gleichermaßen zusammengesetztes Bett von Kristallkeimen (Anteil an der Gesamtmasse ca. 15 %) im Wirbelgerät aufgesprüht. Das Sprühgranulat wird vor der Tablettierung mit dem gleichen Aroma und Magnesium-Stearat vermischt.

### Ergebnisse:

| | Mechan. Mischung | Cogesprühtes Material |
|---|---|---|
| Aussehen | Sorbitpartikel sind vereinzelt erkennbar | homogenes, gut rieselfähiges Tablettiergranulat |
| Tablettierung: Menge: 500 mg Preßkraft 20 kN | | |
| Tablettenhärte: | 20 N | 160 N |
| Abrieb (Friab.) | 22 % | 0,2 % |
| Sensorik | zu weiche Tabletten, rauhe Oberfläche, (nicht akzeptables Ergebnis) | süßsaure Vitamin C-Tablette mit glatter Oberfläche und angenehmen Kaueigenschaften |

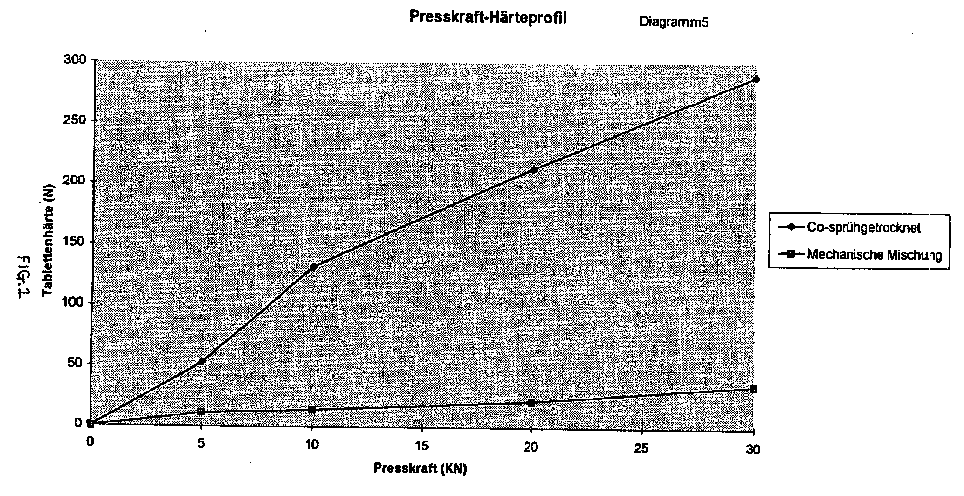

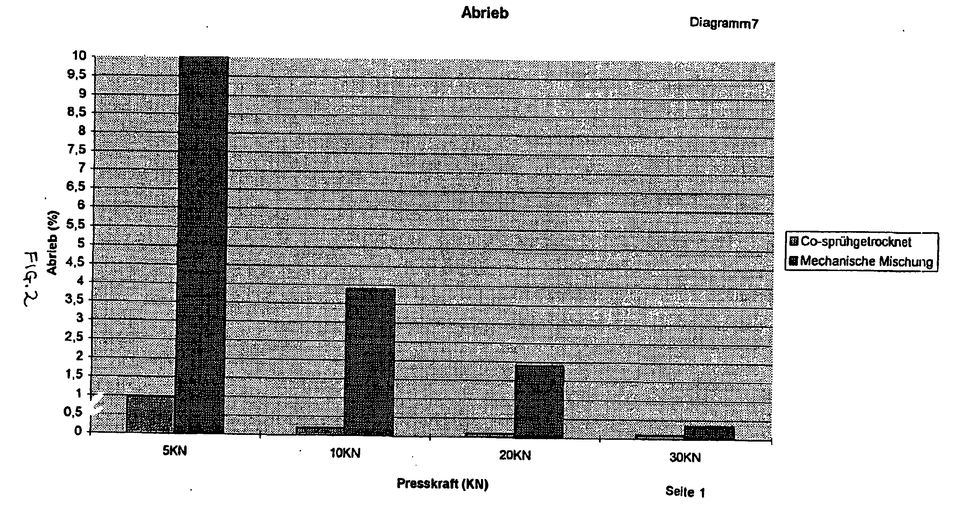

## Patentansprüche

1. Verfahren zur Verbesserung des Geschmacksbildes von festen Formulierungen, welche einen oder mehrere Wirkstoffe enthalten,
**dadurch gekennzeichnet, dass** man eine einen oder mehrere Wirkstoffe enthaltende Zusammensetzung, erhältlich durch Wirbelschichtgranulation,
indem mindestens ein Polyol bzw. Kohllhydrat in Wasser gelöst wird und mindestens ein Wirkstoff in einem Lösungsmittel gelöst oder suspendiert wird und das erhaltene wäßrige Gemisch in einem Luftstrom mit einer Temperatur von 40 bis 120°C verwirbelt wird,
durch direktes Verpressen in einer festen Darreichungsform herstellt.

2. Verfahren gemäß Anspruch 1, **dadurch gekennzeichnet, dass** es sich bei den durch Wirbelschichtgranulation erhaltenen Zusammensetzungen um direkt verpreßbare Wirkstoffformulierungen handelt.

3. Verfahren nach den Ansprüchen 1 - 2, **dadurch gekennzeichnet, dass** man eine Zusammensetzung verwendet, welche
geschmackskorrigierende Substanzen und gegebenenfalls Farbstoffe enthält.

4. Verfahren nach den Ansprüchen 1 - 3, **dadurch gekennzeichnet, dass** man eine Zusammensetzung verwendet, welche einen natürlichen oder synthetischen Süßstoff enthält.

5. Verfahren nach Anspruch 4, **dadurch gekennzeichnet, dass** man eine Zusammensetzung verwendet, welche einen Süßstoff ausgewählt aus der Gruppe Saccharin, Aspartam^{®}, Acesulfam K, Neohesperidin DC, Sucralose, Thaumalin und Steviosid enthält.

6. Verfahren nach den Ansprüchen 1 - 5, **dadurch gekennzeichnet, dass** man eine Zusammensetzung verwendet, welche mindestens einen oder mehrere Polyol(e) ausgewählt aus der Gruppe Sorbit, Mannit, Lactit, Isomalt, Maltit, Erythrit und Xylit enthält.

## Claims

1. Method for improving the taste of solid formulations which comprise one or more active compounds, **characterised in that** a composition comprising one or more active compounds, obtainable by fluidised bed granulation in which at least one polyol or carbohydrate is dissolved in water and at least one active compound is dissolved or suspended in a solvent and the resultant aqueous mixture is fluidised in a stream of air at a temperature of 40 to 120°C, is prepared in a solid administration form by direct compression.

2. Method according to Claim 1, **characterised in that** the compositions obtained by fluidised bed granulation are directly compressible active-compound formulations.

3. Method according to Claims 1 - 2, **characterised in that** a composition is used which comprises flavour-correcting substances and optionally dyes.

4. Method according to Claims 1 - 3, **characterised in that** a composition is used which comprises a natural or synthetic sweetener.

5. Method according to Claim 4, **characterised in that** a composition is used which comprises a sweetener selected from the group saccharin, Aspartame^{®}, acesulfame K, neohesperidin DC, sucralose, thaumalin and stevioside.

6. Method according to Claims 1 - 5, **characterised in that** a composition is used which comprises at least one or more polyol(s) selected from the group sorbitol, mannitol, lactitol, isomalt, maltitol, erythritol and xylitol.

## Revendications

1. Méthode destinée à améliorer le goût de formulations solides comprenant un ou plusieurs composés actifs, **caractérisée en ce qu'**une composition comprenant un ou plusieurs composés actifs, pouvant être obtenue par granulation sur lit fluidisé dans laquelle au moins un polyol ou glucide est solubilisé dans de l'eau et au moins un composé actif est solubilisé ou mis en suspension dans un solvant et le mélange aqueux résultant est fluidisé dans un courant d'air à une température allant de 40 à 120°C, est préparée sous une forme d'administration solide par compression directe.

2. Méthode selon la revendication 1, **caractérisée en ce que** les compositions obtenues par granulation sur lit fluidisé sont des formulations de composé actif directement compressibles.

3. Méthode selon les revendications 1 - 2, **caractérisée en ce qu'**on utilise une composition comprenant des substances de correction d'arôme et éventuellement des colorants.

4. Méthode selon les revendications 1 - 3, **caractérisée en ce qu'**on utilise une composition comprenant un édulcorant naturel ou synthétique.

5. Méthode selon la revendication 4, **caractérisée en ce qu'**on utilise une composition comprenant un édulcorant choisi dans le groupe constitué par la saccharine, l'Aspartame^{®}, l'acésulfame K, la néohespéridine DC, le sucralose, la thaumatine et le stévioside.

6. Méthode selon les revendications 1 - 5, **caractérisée en ce qu'**on utilise une composition comprenant au moins un ou plusieurs polyol(s) choisis dans le groupe constitué par le sorbitol, le mannitol, le lactitol, l'isomalt, le maltitol, l'érythritol et le xylitol.
